# EUROPEAN PATENT SPECIFICATION

(11) **EP 0 538 305 B1**
(45) Date of publication and mention of the grant of the patent: **09.04.1997**
(21) Application number: 91912424.8
(22) Date of filing: 03.07.1991
(51) Int. Cl.: A61K 31/52

(54) **PENCICLOVIR AND FAMCICLOVIR AND RELATED GUANINE DERIVATIVES FOR THE TREATMENT OF THE HIV-1 INFECTIONS**
PENCICLOVIR UND FAMCICLOVIR UND VERWANDTE GUANINDERIVATE ZUR BEHANDLUNG VON HIV-1-INFEKTIONEN
PENCICLOVIR ET FAMCICLOVIR ET DERIVES DE GUANINE APPARENTES UTILISES DANS LE TRAITEMENT D'INFECTIONS PAR LE VIH-1

(30) Priority: 07.07.1990 GB 9015051
(43) Date of publication of application: 28.04.1993
(73) Proprietor: BEECHAM GROUP PLC, Brentford, Middlesex TW8 9EP (GB)
(72) Inventor: KENIG, M. D. J. SmithKline Beecham Pharmaceut., Surrey KT18 5XQ (GB); VERE HODGE, R. A. SmithKline Beecham Pharmaceut., Surrey RH3 7AJ (GB)
(74) Representative: Tocher, Pauline
(86) International application number: GB9101082
(87) International publication number: WO9200742

(56) References cited:
- EP-A- 0 141 927
- EP-A- 0 271 270
- EP-A- 0 306 844
- AT-B- 388 500
- Drugs Future, vol. 15, no. 4, 1990, "BRL-39123" & "BRL-42810", pages 394-395

## Description

This invention relates to the use of compounds in the preparation of a medicament for use in the treatment of HIV-1 infection in humans and animals.

EP-A-141927 (Beecham Group p.l.c.) discloses penciclovir, the compound of formula (A): 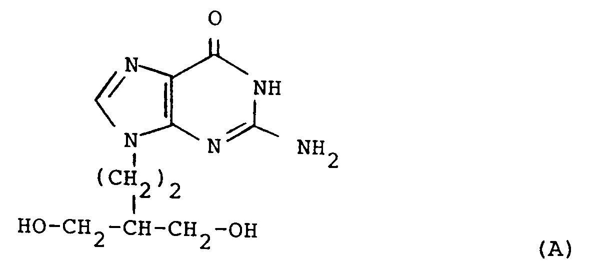 and salts, phosphate esters and acyl derivatives thereof, as antiviral agents. The sodium salt hydrate of penciclovir is disclosed in EP-A-216459 (Beecham Group p.l.c.).
Penciclovir and its antiviral activity is also disclosed in Abstract P.V11-5 p.193 of 'Abstracts of 14th Int. Congress of Microbiology', Manchester, England 7-13 September 1986 (Boyd et. al.).

Pro-drugs of the compound of formula (A) are of formula (B): 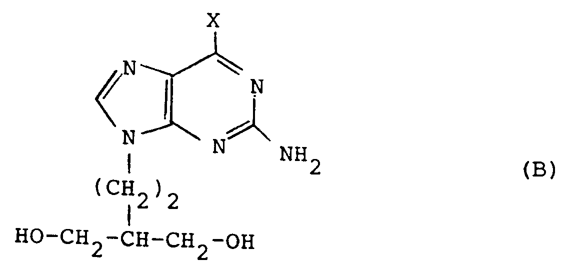 and salts and derivatives thereof as defined under formula (A); wherein X is C₁₋₆ alkoxy, NH₂ or hydrogen. The compounds of formula (B) wherein X is C₁₋₆ alkoxy or NH₂ are disclosed in EP-A-141927 and the compounds of formula (B) wherein X is hydrogen, disclosed in EP-A-182024 (Beecham Group p.l.c.) are preferred prodrugs. A particularly preferred example of a compound of formula (B) is that wherein X is hydrogen and wherein the two OH groups are in the form of the acetyl derivative, described in Example 2 of EP-A-182024, hereinafter referred to as famciclovir.

The compounds of formulae (A) and (B) and salts and derivatives thereof have been described as useful in the treatment of infections caused by herpesviruses, such as herpes simplex type 1, herpes simplex type 2, varicella-zoster and Epstein-Barr viruses.

It has now been discovered that these compounds have potential activity against the human immunodeficiency virus (HIV-1), in patients also infected with herpesviruses, and are therefore of potential use in the treatment of HIV infections in such patients.

This discovery is related to the ability of the triphosphate derivative of penciclovir to inhibit the RNA-directed DNA polymerase (reverse transcriptase) activity of human immunodeficiency virus type 1 (HIV-1). The reverse transcriptase of HIV-1 is a virus-encoded enzyme essential for the conversion of genomic RNA into proviral ds-DNA, and is therefore an excellent molecular target for antiviral chemotherapy.

The ability of HIV to enter cells previously infected with herpesviruses is known (for example, B-lymphocytes infected with EBV¹). The presence of both herpes and human immunodeficiency viruses in the same cell has particular consequences.
1. Penciclovir would be phosphorylated by herpes virus-encoded thymidine kinase leading to a high level of penciclovir triphosphate². The triphosphate formed is not only an inhibitor of herpes DNA polymerase, but this work indicates that it also inhibits HIV reverse transcriptase.
2. HIV replication may be enhanced by herpesvirus transactivating factors. A product of HSV, ICP-4 (infected-cell protein) can increase the initiation of HIV transcription.
3. Double infection of herpesviruses and HIV may result in phenotypic mixing and the production of 'pseudotype' HIV particles bearing herpesvirus envelope glycoproteins³. The packaging of HIV genomic RNA with HSV capsid proteins is also believed to occur. Either situation may lead to the infection by HIV of CD4-negative, herpesvirus-permissible cells, previously not susceptible to entry of this virus. This may also result in doubly-infected cells.

Accordingly, the present invention provides Use of a compound of formula (A): 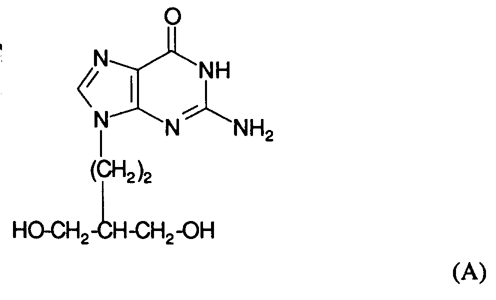 or a pro-drug of formula (B): 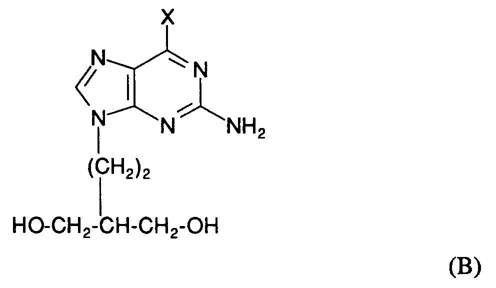 wherein X is C₁₋₆ alkoxy, NH₂ or hydrogen;
or a pharmaceutically acceptable salt, phosphate ester and/or acyl derivative of either of the foregoing.
in the manufacture of a medicament for use in the treatment of HIV-1 infections in mammals, which mammals are infected with herpesviruses.

The term 'acyl derivative' is used herein to include any derivative of the compounds of formula (A) in which one or more acyl groups are present. Such derivatives are included as pro-drugs of the compounds of formula (A) in addition to those derivatives which are per se biologically active.

Examples of pro-drugs, pharmaceutically acceptable salts and derivatives are as described in the aforementioned European Patent references, the subject matter of which are incorporated herein by reference.

A particular pro-drug of interest is 9-(4-acetoxy-3-acetoxymethylbut-1-yl)-2-aminopurine, known as famciclovir.

The compound of formula (A) may also be in one of the forms disclosed in EP-A-216459 (Beecham Group p.l.c.).

The compound of formula (A), pro-drugs, salts and derivatives may be prepared as described in the aforementioned European Patent references.

The compound, in particular, famciclovir, may be administered by the oral route to humans and may be compounded in the form of syrup, tablets or capsule. When in the form of a tablet, any pharmaceutical carrier suitable for formulating such solid compositions may be used, for example magnesium stearate, starch, lactose, glucose, rice, flour and chalk. The compound may also be in the form of an ingestible capsule, for example of gelatin, to contain the compound, or in the form of a syrup, a solution or a suspension. Suitable liquid pharmaceutical carriers include ethyl alcohol, glycerine, saline and water to which flavouring or colouring agents may be added to form syrups.

For parenteral administration, fluid unit dose forms are prepared containing the compound and a sterile vehicle. The compound depending on the vehicle and the concentration, can be either suspended or dissolved. Parenteral solutions are normally prepared by dissolving the compound in a vehicle and filter sterilising before filling into a suitable vial or ampoule and sealing. Advantageously, adjuvants such as a local anaesthetic, preservatives and buffering agents are also dissolved in the vehicle. To enhance the stability, the composition can be frozen after filling into the vial and the water removed under vacuum.

Parenteral suspensions are prepared in substantially the same manner except that the compound is suspended in the vehicle instead of being dissolved and sterilised by exposure to ethylene oxide before suspending in the sterile vehicle. Advantageously, a surfactant or wetting agent is included in the composition to facilitate uniform distribution of the compound of the invention.

Preferred parenteral formulations include aqueous formulations using sterile water or normal saline, at a pH of around 7.4 or greater, in particular, containing penciclovir sodium salt hydrate.

As is common practice, the compositions will usually be accompanied by written or printed directions for use in the medical treatment concerned.

An amount effective to treat the virus infection depends on the nature and severity of the infection and the weight of the mammal.

A suitable dosage unit might contain from 50mg to 1g of active ingredient, for example 100 to 500mg. Such doses may be administered 1 to 4 times a day or more usually 2 or 3 times a day. The effective dose of compound will, in general, be in the range of from 0.2 to 40mg per kilogram of body weight per day or, more usually, 10 to 20 mg/kg per day.

The present invention also provides the use of a compound of formula (A) or a pro-drug, or a pharmaceutically acceptable salt, phosphate ester and/or acyl derivative of either of the foregoing, in the preparation of a medicament for use in the treatment of HIV-1 infections in mammals, including humans, which mammals are infected with herpesviruses. Such treatment may be carried out in the manner as hereinbefore described.

The present invention further provides a pharmaceutical composition for use in the treatment of HIV-1 infections in mammals, including humans, which mammals are infected with herpesviruses, which comprises an effective amount of a compound of formula (A) or a pro-drug, or a pharmaceutically acceptable salt, phosphate ester and/or acyl derivative of either of the foregoing, and a pharmaceutically acceptable carrier. Such compositions may be prepared in the manner as hereinafter described.

The compound of formula (A) and its prodrugs show a synergistic antiviral effect in conjunction with interferons; and treatment using combination products comprising these two components for sequential or concomitant administration, by the same or different routes, are therefore within the ambit of the present invention. Such products are described in EP-A-271270 (Beecham Group p.l.c.).

It will be appreciated that the treatment of herpesvirus infected patients may include prophylaxis of herpesvirus episode attacks (suppressive treatment). In patients with HSV infection only, suppressive treatment would probably only be given to those patients with frequent recurrences. In contrast, the aforementioned finding in relation to HIV-1, indicates the need for continuous suppressive treatment with penciclovir to all HIV-1 infected patients with herpesvirus recurrences, particularly HSV-1, HSV-2 and VZV recurrences, even though these recurrences may be infrequent.

The following biochemical data illustrate the invention.

### BIOCHEMICAL DATA

### Materials and Methods

Chemicals [³H]dGTP (16.3 Ci/mmol) was purchased from Amersham International, Aylesbury, U.K. Template primer Poly (rC). p(dG)₁₂₋₁₈ (1:1, rC:dG ratio) and 2',3'-dideoxyguanosine-5-triphosphate (ddGTP) were obtained from Pharmacia Ltd., Milton Keynes, U.K. Penciclovir triphosphate (PCV-TP) was prepared in the laboratories of SmithKline Beecham Pharmaceuticals, Great Burgh, United Kingdom.

Reverse transcriptase Purified, E. coli expressed HIV-1 reverse transcriptase (RT) was supplied by the Protein Biochemistry Department of SmithKline Beecham Pharmaceuticals, Upper Merion, U.S.A. The enzyme was stored and diluted in a buffer containing 50mM Tris-HCl (pH 8.0), 10mM Hepes, 110mM NaCl, 5.7mM DTT, 0.3mM EDTA, 0.06% Triton X-100, 50% glycerol.

Assay for reverse transcriptase activity The reaction mixture for the HIV-1 RT assay contained in a volume of 120µl: 33mM Tris HCl (pH 8.0), 100mM KCl, 3.3mM MgCl₂, 3.3mM dithiothreitol, 0.2mM glutathione, 0.33mM EGTA (ethylene glycol-bis-(β-aminoethyl ether) N,N-tetra acetic acid), 0.033% Triton X-100, 1.02µM [³H]dGTP, 0-12.39µM inhibitors ddGTP, PCV-TP, 0.3 A₂₆₀ units/ml Poly (rC). p(dG)₁₂₋₁₈ and 167ng/ml RT (equivalent to 2.85nM for an equimolar mixture of p66 and p51 polypeptides). The reaction mixtures without RT were prepared in the microwells of a 96-well plate and preincubated at 37°C before the reactions were started by the addition of 20µl of the enzyme solution. The plates were then incubated for 65 minutes at 37°C. The incorporation rate was linear for the uninhibited control reaction over this time period. The reactions were terminated by the addition of 40µl of EDTA solution (0.2M, pH7.0). The individual reaction mixtures were transferred to a DEAE filter mat (1205-405, LKB Wallac, Finland), prewetted with 0.3M NaCl/0.03M Na citrate, using a cell harvester (1295-001, LKB Wallac). The filter mat was washed three times in the NaCl/Na citrate buffer and then once in 95% ethanol. Scintillation fluid (Beta Plate Scint, LKB Wallac) was added to the dried filter, and the reaction mixtures assayed for incorporation of radioactive dGMP in an LKB 1205 Beta Plate Liquid Scintillation Counter.

### Results

The counts per minute obtained with the uninhibited control reaction mixture, and with a range of concentrations of ddGTP and PCV-TP, are shown in Tables 1 and 2 respectively. From the plots of % inhibition against concentration of inhibitor, approximate IC₅₀ values were obtained as follows:-

| | |
|---|---|
| ddGTP: | 25nM |
| (R/S)PCV-TP: | 4.3µM |

### Conclusion

These results indicate that the concentration of penciclovir triphosphate required to give 50% inhibition of HIV-1 reverse transcriptase is approximately 4µM. This level of PCV-TP should be obtained in the herpes-infected cell².

**Table 1**

| Inhibition of HIV-1 Reverse Transcriptase by ddG-Triphosphate | | |
|---|---|---|
| Concentration of ddGTP (nM) | Incorporation of dGMP (c.p.m.) | % Inhibition |
| 0 | 117892 | |
| 1 | 119726 | 0 |
| 10 | 89949 | 23.7 |
| 25 | 58703 | 50.2 |
| 50 | 35091 | 70.2 |
| 75 | 26132 | 77.8 |
| 100 | 22161 | 81.2 |

**Table 2**

| Inhibition of HIV-1 Reverse Transcriptase by PCV-Triphosphate | | |
|---|---|---|
| Concentration of PCV-TP (µM) | Incorporation of dGMP (c.p.m.) | % Inhibition |
| 0 | 88892 | |
| 0.124 | 85313 | 4.0 |
| 1.24 | 70061 | 21.2 |
| 3.10 | 51866 | 41.7 |
| 6.19 | 32921 | 63.0 |
| 9.29 | 26436 | 70.3 |
| 12.39 | 23844 | 73.2 |

### References

1. Complement Receptor 2 Mediates Enhancement of Human Immunodeficiency virus infection in Epstein-Barr virus-carrying B cells.
   Tremblay et al., J. Exp. Med. 171, 1791 (1990).
2. Mode of action of 9-(4-hydroxy-3-hydroxymethylbut-1-yl)guanine (BRL 39123) against herpes simplex virus in MRC-5 cells.
   Vere Hodge and Perkins, A.A.C., 33, 223 (1989).
3. Phenotypic mixing between human immunodeficiency virus and vesicular stomatitis virus or herpes simplex virus.
   Zhu et al., J. of AIDS, 3, 215 (1990).

## Claims

1. Use of a compound of formula (A): 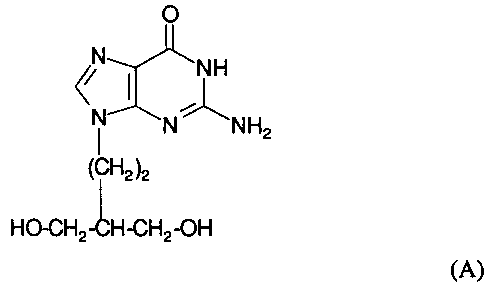 or a pro-drug of formula (B): 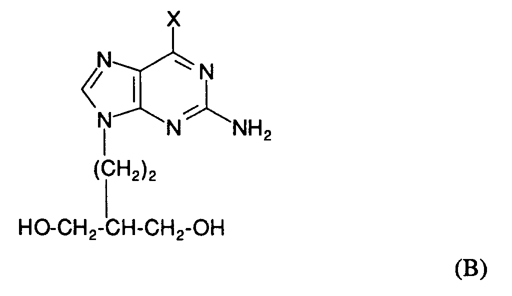 wherein X is C₁₋₆ alkoxy, NH₂ or hydrogen;
or a pharmaceutically acceptable salt, phosphate ester and/or acyl derivative of either of the foregoing.
in the manufacture of a medicament for use in the treatment of HIV-1 infections in mammals, which mammals are infected with herpesviruses.

2. A use according to claim 1 wherein the compound is the compound of formula (A).

3. A use according to claim 1 wherein the compound is the sodium salt hydrate of the compound of formula (A).

4. A use according to claim 3 wherein the medicament is in an aqueous formulation, adapted for parenteral administration.

5. A use according to claim 1 wherein the compound is a pro-drug of formula (B): 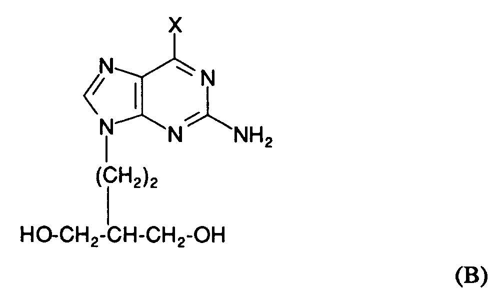 wherein X is C₁₋₆ alkoxy, NH₂ or hydrogen, or a salt or derivative thereof, as defined in claim 1.

6. A use according to claim 5 wherein the pro-drug compound of formula (B) is wherein X is hydrogen, or a pharmaceutically acceptable salt, phosphate ester and/or acyl derivative of either of the foregoing.

7. A use according to claim 6 wherein the pro-drug compound of formula (B) is famciclovir, wherein X is hydrogen and wherein the two OH groups are in the form of the acetyl derivative.

8. A use according to claim 7 wherein the medicament is adapted for oral administration.

9. A use according to claim 1 wherein the compound administered is in a 50mg to 1g unit dose.

10. Use of penciclovir, or a pro-drug therefor as defined in claim 1, in the manufacture of a medicament for use in continuous suppressive treatment of HIV infected patients with HSV or VZV recurrences.

## Patentansprüche

1. Verwendung einer Verbindung der Formel (A): 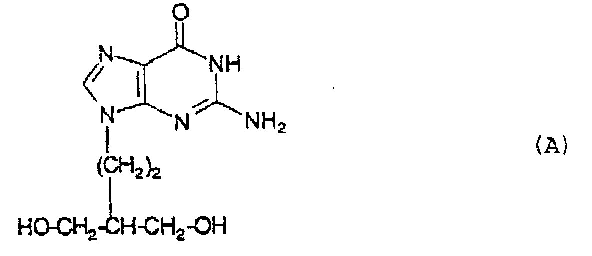 oder eines Pro-Drugs der Formel (B): 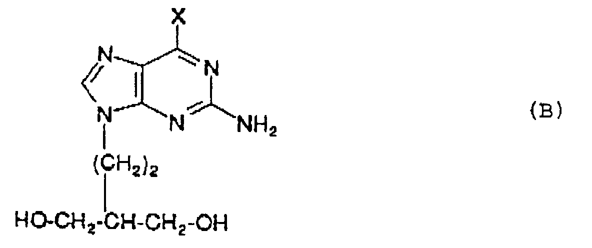 in der X ein C₁₋₆-Alkoxyrest, eine NH₂-Gruppe oder ein Wasserstoffatom ist; oder
eines pharmazeutisch verträglichen Salzes, eines Phosphatesters und/oder eines Acylderivats einer der vorstehenden Verbindungen
für die Herstellung eines Medikamente zur Verwendung bei der Behandlung von HIV-1-Infektionen in Säugern, wobei die Säuger mit Herpesviren infiziert sind.

2. Verwendung nach Anspruch 1, wobei die Verbindung die Verbindung der Formel (A) ist.

3. Verwendung nach Anspruch 1, wobei die Verbindung das Natriumsalzhydrat der Verbindung der Formel (A) ist.

4. Verwendung nach Anspruch 3, wobei das Medikament in einer wäßrigen Formulierung vorliegt, die für eine parenterale Verabreichung angepaßt ist.

5. Verwendung nach Anspruch 1, wobei die Verbindung ein Pro-Drug der Formel (B) ist, 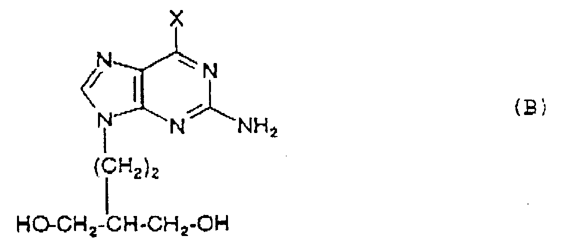 in der X ein C₁₋₆-Alkoxyrest, eine NH₂-Gruppe oder ein Wasserstoffatom ist, oder ein Salz oder Derivat davon gemäß der Definition in Anspruch 1.

6. Verwendung nach Anspruch 5, wobei die Pro-Drug-Verbindung der Formel (B) die Verbindung ist, in der X ein Wasserstoffatom ist, oder ein pharmazeutisch verträgliches Salz, ein Phosphatester und/oder ein Acylderivat einer der vorstehenden Verbindungen.

7. Verwendung nach Anspruch 6, wobei die Pro-Drug-Verbindung der Formel (B) Famciclovir ist, wobei X ein Wasserstoffatom ist und wobei die zwei OH-Gruppen in Form des Acetylderivats vorliegen.

8. Verwendung nach Anspruch 7, wobei das Medikament für die orale Verabreichung angepaßt ist.

9. Verwendung nach Anspruch 1, wobei die verabreichte Verbindung in einer Einheitsdosis von 50 mg bis 1 g vorliegt.

10. Verwendung von Penciclovir oder eines Pro-Drugs davon gemäß der Definition in Anspruch 1 für die Herstellung eines Medikaments zur Verwendung bei einer fortlaufenden suppressiven Behandlung von HIV-infizierten Patienten mit HIV- oder VZV-Rezidiven.

## Revendications

1. Utilisation d'un composé de formule (A) : 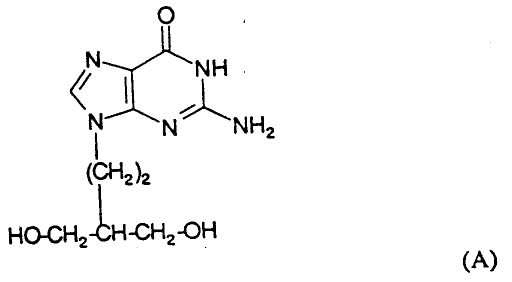 ou d'une prodrogue de formule (B) : 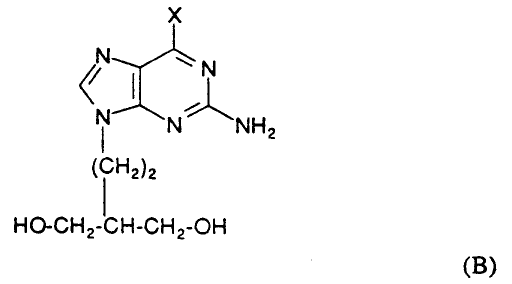 où X est un groupe alcoxy en C₁₋₆, NH₂ ou un atome d'hydrogène ;
ou d'un sel, phosphate ester et/ou dérivé acyle acceptable du point de vue pharmaceutique de l'un ou l'autre des composés précédents ;
dans la fabrication d'un médicament utile dans le traitement d'infections par VIH-1 chez des mammifères, lesquels mammifères sont infectés par des virus de la famille des herpès.

2. Utilisation suivant la revendication 1, dans laquelle le composé est le composé de formule (A).

3. Utilisation suivant la revendication 1, dans laquelle le composé est le sel sodique hydraté du composé de formule (A).

4. Utilisation suivant la revendication 3, dans laquelle le médicament est dans une formulation aqueuse adaptée pour l'administration par voie parentérale.

5. Utilisation suivant la revendication 1, dans laquelle le composé est une prodrogue de formule (B) : 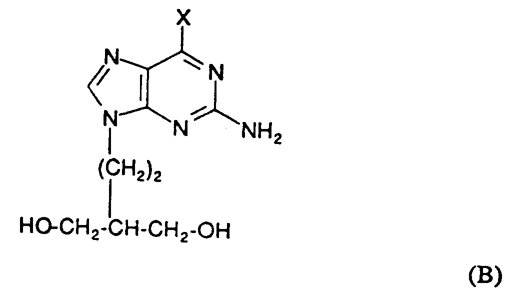 dans laquelle X est un groupe alcoxy en C₁₋₆, NH₂ ou un atome d'hydrogène ou un sel ou un dérivé de celui-ci tel que défini dans la revendication 1.

6. Utilisation suivant la revendication 5, dans laquelle la prodrogue de formule (B) est un composé dans lequel X est un atome d'hydrogène, ou un sel, un phosphate ester et/ou un dérivé acyle acceptable du point de vue pharmaceutique de celui-ci.

7. Utilisation suivant la revendication 6, dans laquelle la prodrogue de formule (B) et le famciclovir, dans laquelle X est un atome d'hydrogène et dans les deux groupes OH sont sous la forme du dérivé acétyle.

8. Utilisation suivant la revendication 7, dans laquelle le médicament est adapté pour l'administration par voie orale.

9. Utilisation suivant la revendication 1, dans laquelle le composé à administrer est dans une dose unitaire de 50 mg à 1 g.

10. Utilisation de penciclovir ou d'une prodrogue de celui-ci suivant la revendication 1, dans la fabrication d'un médicament utile dans un traitement super-continu de patients infectés par un VIH avec récurrences de HSV ou VZV.
